# EUROPÄISCHE PATENTANMELDUNG

(11) **EP 1 695 670 A1**
(43) Veröffentlichungstag der Anmeldung: **30.08.2006**
(21) Anmeldenummer: 05004032.8
(22) Anmeldetag: 24.02.2005
(51) Int. Cl.: A61B 18/20, A61B 19/00

(54) **Tragbare Laserprojektionsvorrichtung für die medizintechnische Bilddarstellung**

(71) Anmelder: BrainLAB AG, 85551 Kirchheim/Heimstetten (DE)
(72) Erfinder: Schmidt, Robert, 81371 München (DE); Frielinghaus, Nils, 85551 Heimstetten (DE); Rossner, Holger-Claus, 85622 Feldkirchen (DE); Seifert, Uli, 85570 Markt Schwaben (DE); Pfäffle, Alexander, 85435 Erding (DE)
(74) Vertreter: Schwabe - Sandmair - Marx

(57) **Zusammenfassung**

Die Anmeldung betrifft ein medizintechnisches Bilddarstellungssystem mit:
a) einem Bildspeicherungs- und Bildverarbeitungseinrichtung (15), die Patientenkörper-Bilddaten und/oder Bildzusatzdaten speichert, verarbeitet und ausgibt; und mit
b) einer tragbaren Laserprojektionsvorrichtung (10), welche die von der Bildspeicherungs- und Bildverarbeitungseinrichtung ausgegebenen Bilddaten und/oder Bildzusatzdaten auf einen Projektionsort projiziert.
Sie betrifft ferner ein medizintechnisches Bilddarstellungsverfahren, bei dem eine Bildspeicherungs- und Bildverarbeitungseinrichtung (15) Patientenkörper-Bilddaten und/oder Bildzusatzdaten speichert, verarbeitet und ausgibt; und bei dem mit einer tragbaren Laserprojektionsvorrichtung (10) die von der Bildspeicherungs- und Bildverarbeitungseinrichtung ausgegebenen Bilddaten und/oder Bildzusatzdaten auf einen Projektionsort projiziert werden.

## Beschreibung

Die vorliegende Erfindung betrifft ein medizintechnisches Bilddarstellungssystem. Solche medizintechnischen Bilddarstellungssysteme werden verwendet, um Ärzte bei der Diagnose und der Therapie, insbesondere der Chirurgie zu unterstützen. Sie verarbeiten Patientendaten, die vorab mit Bilderfassungsgeräten akquiriert wurden, nämlich beispielsweise mit Schichtbildaufnahmeverfahren (CT-, MR-Scanner) oder mit Projektionsverfahren wie Röntgendurchleuchtung.

Die Patientenkörper-Bilddaten und zusätzliche Daten, beispielsweise zur Behandlungsplanung, werden Ärzten und Behandlungspersonal herkömmlicherweise mit Hilfe von Bildschirmanzeigen zugänglich gemacht.

Ein System mit Bildschirmdarstellung ist beispielsweise aus der DE 19 639 615 C2 bekannt.

Solche oben genannten Systeme, die Bildschirmdarstellungen verwenden, haben eigentlich immer den Nachteil, dass man den relativ großen Bildschirm entweder nur stationär bereitstellt, so dass der Arzt dauernd von seinem Arbeitsgebiet aufblicken muss, um die Informationen auf dem Monitor abzulesen. In anderen Fällen wird der Monitor an relativ komplizierten Gelenkarmen befestigt, um ihn zumindest in die Nähe des Sichtfeldes des Operateurs bringen zu können. Solche Konstruktionen sind aber teuer, und außerdem ist der Monitor oft auch im Weg.

Eine andere Methode medizintechnischer Bilddarstellung ist die Projektion. Aus den US-Patenten Nr. 5,715,836; 6,317,616 und 6,314,311 sind beispielsweise Projektionssysteme für Patienten- oder Eingriffsplanungsdaten bekannt, jedoch werden hier komplizierte Projektionsvorrichtungen verwendet, deren Einsatz sich nicht überall anbietet oder nicht ohne großen Aufwand zusätzlich realisieren lässt.

Es ist die Aufgabe der vorliegenden Erfindung, ein medizintechnisches Bilddarstellungssystem bereitzustellen, welches die oben genannten Nachteile des Standes der Technik überwindet. Insbesondere soll ein in einfacher Weise und speziell auch zusätzlich bereitstellbares Bilddarstellungssystem vorgeschlagen werden, das eine einfache Handhabung gestattet.

Diese Aufgabe wird erfindungsgemäß durch ein medizintechnisches Bilddarstellungssystem und ein Bilddarstellungsverfahren gemäß den Ansprüch 1 und 7 gelöst. Die Unteransprüche definieren bevorzugte Ausführungsformen der Erfindung.

Gemäß der vorliegenden Erfindung weist ein medizintechnisches Bilddarstellungssystem zwei Hauptkomponenten auf, nämlich eine Bildspeicherungs-und Bildverarbeitungseinrichtung, die Patientenkörper-Bilddaten und/oder Bildzusatzdaten speichert, verarbeitet und ausgibt. Die zweite Komponente ist eine tragbare Laserprojektionsvorrichtung, welche die von der Bildspeicherungs- und Bildverarbeitungseinrichtung ausgegebenen Bilddaten und/oder Bildzusatzdaten auf einen Projektionsort projiziert. Die Bilddaten sind hierbei angepasste und anwendungsgerecht dargestellte Daten über die Patientenanatomie, die meist vorab aus Schichtbildaufnahmen oder Projektions-Durchleuchtungsaufnahmen gewonnen werden. Bilddaten können auch aus Computergenerierten Daten gewonnen werden (z.B. aus Videodaten die während eines Eingriffs in Position und Lage gespeichert werden). Bildzusatzdaten umfassen alle möglichen Daten, welche die Eingriffsplanung oder Eingriffsunterstützung zum Thema haben können. Beispielsweise können geplante Instrumententrajektorien angezeigt werden, ebenso wie die tatsächlichen georteten Instrumente. Intuitive Darstellungen (Pfeile, Tunneldarstellungen, konzentrische Kreise) sind möglich, um den Arzt bei der Behandlung anzuleiten. All diese Daten können die genannten Bildzusatzdaten sein.

Wenn hierin von einer "tragbaren Laserprojektionsvorrichtung" gesprochen wird, so ist damit z.B. eine solche Laserprojektionsvorrichtung gemeint, die nicht unbedingt und wie herkömmlich stationär angeordnet werden muss, um ihre Funktion zu erfüllen. Sie kann (frei) handhabbar sein, d.h. mit der Hand gehalten und ausgerichtet werden, oder sie kann auch in einem solchen Sinne tragbar sein, dass sie von einem sich bewegenden oder bewegten medizinischen Instrument getragen wird. Dabei ist es bevorzugt, dass tatsächlich die Projektionsvorrichtung selbst in diesem Sinne tragbar ist, d.h. bevorzugt wird nicht lediglich eine "tragbare" Bildumlenkeinheit bereitgestellt.

Der Vorteil gegenüber dem oben zuerst angesprochenen Bildschirm- bzw. Monitor-Bilddarstellungssystemen liegt schon ganz einfach darin, dass man sich den Bildschirm bzw. Monitor sparen kann. Laserprojektionsvorrichtungen der neueren Generationen sind dazu in der Lage, monochrome aber auch schon farbige Projektionen am Projektionsort zu erstellen, die Monitorbildern in der Qualität nicht nachstehen, und dies bei einer Miniaturisierung (Miniator-Laserprojektor), die es gestattet, die Projektionsvorrichtung zwischen zwei Fingern einer Hand zu halten. Der Arzt muss nicht mehr auf einen stationär angeordneten Monitor sehen oder einen Monitor da anordnen, wo er zwar gut sichtbar, aber möglichst wenig im Wege ist, sondern er kann sich ohne Weiteres mit Hilfe der tragbaren Laserprojektionsvorrichtung das gewünschte Bild dorthin projizieren lassen, wo es gerade am bequemsten für ihn sichtbar ist. Die Projektionsvorrichtung kann er dabei vorübergehend selbst halten oder durch einen Assistenten halten lassen; der Vorteil ist immer, dass er das Bild unmittelbar und schnell dorthin bringen kann, wo er es gerade braucht. Wegen der Tragbarkeit der Laserprojektionsvorrichtung ist diese auch den üblichen Projektionssystemen überlegen, da die Projektionen, beispielsweise Projektionen auf den Körper, ohne Weiteres aus verschiedenen Richtungen und Stellungen erfolgen können, ohne dass der Arzt bei seiner Tätigkeit behindert wird und ohne dass die Tätigkeit des Arztes die Projektion beeinträchtigt (beispielsweise wenn er in den Projektionsstrahl hineinkommt).

Die Bildspeicherungs- und Bildverarbeitungseinrichtung kann ein Teil eines medizintechnischen Navigationssystems sein oder einem medizintechnischen Navigationssystem zugeordnet sein. Solche Navigationssysteme sind bekannt, sie speichern Patienten-Körperbilddaten und orten Instrumente, damit eine Zuordnung der tatsächlichen Instrumentenposition zu den Körperdaten erfolgen kann. Die Navigationssysteme können, da sie die Bilddaten ohnehin schon meist vorliegen haben, sehr gut im Rahmen der vorliegenden Erfindung als Bildspeicher oder Bildverarbeitungseinheit zum Einsatz kommen. Wenn hier von Bildausgabe die Rede ist, so ist im Rahmen des Navigationssystems damit gemeint, dass die Bilddaten beispielsweise als ein Datenoutput zur Verfügung gestellt werden, im Gegensatz zur tatsächlichen Bilddarstellung (Monitor, Projektion).

Wenn ein medizintechnisches Navigationssystem verwendet wird, so bietet es sich im Rahmen der vorliegenden Erfindung auch an, die tragbare Laserprojektionsvorrichtung mit einer Navigationsreferenz auszustatten, die von dem Navigationssystem geortet und positionell bestimmt werden kann. Das ins System eingebundene Navigationssystem kann damit feststellen, wo die Laserprojektionsvorrichtung sich gerade befindet. Dies hat den Vorteil, dass die Laserprojektion also die Bilddarstellung selbst an die Position der Projektionsvorrichtung angepasst werden kann, was sich insbesondere dann positiv auswirkt, wenn Teile der Patientenanatomie oder Eingriffsplanungsdaten auf den Patientenkörper selbst projiziert werden. So kann z.B. die Richtung des vorgeschlagenen Einschnitts immer richtig auf einen navigierten Patientenkörperteil aufprojiziert werden, wenn die Projektionsvorrichtung selbst ebenfalls navigiert, d.h. mittels der Navigationsreferenz geortet wird.

In einer weiteren Ausgestaltung würde dann die Laserprojektionsvorrichtung an einem medizinischen Instrument angeordnet oder in ein medizinisches Instrument integriert. In diesem Sinne ist das Wort "tragbar" so zu verstehen, dass das Instrument die Laserprojektionsvorrichtung trägt. Es lässt sich damit unmittelbar am Behandlungsort Bild- und Navigationsunterstützung bereitstellen, und zwar gerade für das Instrument, das in diesem Moment verwendet wird. Dabei ist es von Vorteil, wenn das medizinische Instrument selbst auch eine Navigationsreferenz aufweist, die von dem Navigationssystem geortet und positionell bestimmt werden kann. Bei einer starren Verbindung von Instrument und Projektor genügt eine Navigationsreferenz.

Gemäß einer Ausführungsform kann die Laserprojektionsvorrichtung, insbesondere abnehmbar, an einem medizinischen Behandlungsgerät oder einem behandlungsunterstützenden Gerät angeordnet sein oder angeordnet werden, insbesondere an einem der folgenden Geräte:
- einem chirurgischen Mikroskop;
- einem Bilderfassungsgerät, z.B. einem Schichtbilderfassungsgerät oder einem C-Bogen-Röntgengerät oder Fluoroskop;
- einem Strahlenbehandlungsgerät (LINAC);
- einer Operationslampe;
- einem Pointer,
- einem invasiven Instrument, z. B. einem Skalpell;
- einer Biopsie-Nadelhaltevorrichtung;
- einer Biopsienadel.

Die Erfindung betrifft ferner ein medizintechnisches Bilddarstellungsverfahren, bei dem eine Bildspeicherungs- und Bildverarbeitungseinrichtung Patientenkörper-Bilddaten und/oder Bildzusatzdaten speichert, verarbeitet und ausgibt und bei dem mit einer tragbaren Laserprojektionsvorrichtung die von der Bildspeicherungs- und Bildverarbeitungseinrichtung ausgegebenen Bilddaten und/oder Bildzusatzdaten auf einen Projektionsort projiziert werden.

Dieses Bilddarstellungsverfahren hat natürlich die oben schon beschriebenen Vorteile gegenüber der herkömmlichen Bilddarstellung in der Medizintechnik. Es können Bilddaten und/oder Bildzusatzdaten projiziert werden, die aus einem medizintechnischen Navigationssystem stammen, wobei die Bilddaten Patientenanatomiedaten sein können, insbesondere Projektionen aus Schichtbildaufnahmen und Durchleuchtungsaufnahmen. Die Bildzusatzdaten können Zusatzinformationen zu den Bilddaten und/oder Hilfsdarstellungen umfassen, wie Navigationsanweisungen in Pfeilform oder als Richtungsanzeigen bzw. Ausrichtungsanweisungen, aber genauso Umrisse von Strukturen oder 3D Objekte.

Bei einer Ausführungsform des erfindungsgemäßen Bilddarstellungsverfahrens werden die Bilddaten und/oder die Bildzusatzdaten durch die Laserprojektionsvorrichtung, die in einem medizinischen Instrument integriert oder daran angeordnet sein kann, auf ein Patientenkörperteil projiziert, wobei sowohl der Patientenkörperteil als auch die Laserprojektionsvorrichtung bzw. das Instrument von dem Navigationssystem geortet und positionell bestimmt werden.

Die Laserprojektionsvorrichtung kann in Kombination mit dem Navigationssystem als eine, insbesondere dynamische, Benutzerschnittstelle verwendet werden, insbesondere als virtuelle Eingabeeinheit. Auch kann sie einem Videokamera-Trackingsystem zugeordnet werden und die von diesem System erfassten Bildinformationen ausgeben.

Sie kann ferner beispielsweise verwendet werden, um auf eine Körperoberfläche ein bekanntes und diversifizierendes Muster aufzuprojizieren, wobei das aufprojizierte Muster von einer Videokamera aufgenommen bzw. (re-)detektiert wird, um aus der Form oder Verformung des Musters zu Registrierungszwecken auf die Form der Körperoberfläche zu schließen. Das Muster wäre dann im Sinne der oben schon beschriebenen "Bildzusatzdaten" einzuordnen.

Die Erfindung wird im Weiteren anhand von bevorzugten Ausführungsformen und unter Bezugnahme auf die beiliegenden Zeichnungen näher erläutert. Sie kann sämtliche hierin aufgeführten Merkmale einzeln oder in jedweder Kombination umfassen. In den Zeichnungen zeigen:
- Figur 1: eine erfindungsgemäße medizinische Bilddarstellung mit einem nicht navigierten Miniatur-Laserprojektor;
- Figur 2: eine erfindungsgemäße medizintechnische Bilddarstellung mit einem navigierten Miniatur-Laserprojektor; und
- Figur 3: einen Miniatur-Laserprojektor an einem chirurgischen Instrument mit projizierter Instrumentenführungshilfe.

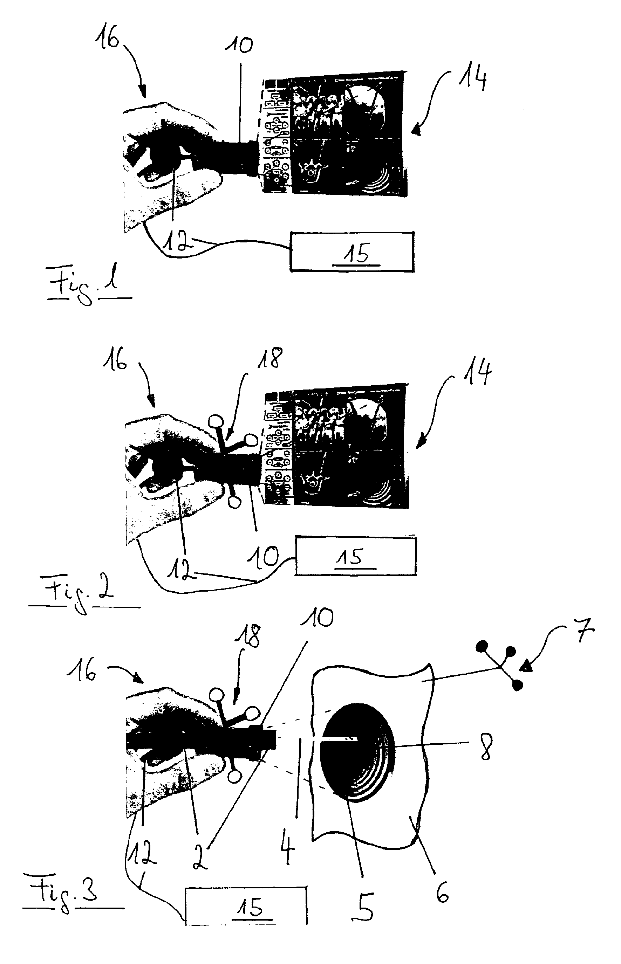

Eine einfache Ausführungsform der vorliegenden Erfindung ist in der Figur 1 gezeigt. In dieser Figur 1 ist ein tragbarer Miniatur-Laserprojektor mit dem Bezugszeichen 10 versehen worden. Er kann leicht mit einer Hand 16 eines Bedieners gehalten werden, und er erhält Daten über die Datenleitung 12 von einer Bildspeicherungs- und Bildverarbeitungseinrichtung 15. Die Einrichtung 15 kann ein Navigationssystem sein, welches die notwendigen, darzustellenden Bilddaten und Bildzusatzdaten liefern kann. Zwischen Navigationssystem 15 und Laserprojektor 10 kann sich noch eine nicht dargestellte Einrichtung befinden, welche für die Lichtstrahlerzeugung verantwortlich ist. Es ist aber auch möglich, alle notwendigen Systeme in das Navigationssystem 15 zu integrieren, so dass von diesem aus nur eine einzige Leitung 12 zum Projektor 10 führt. Das Navigationssystem 15 umfasst auch alle anderen (nicht dargestellten) Untersysteme, die Navigationssystemen üblicherweise eigen sind, also z.B. Trackingkameras, Referenzmarker und Referenzmarkeranordnungen, Infrarotlichtquellen, usw.

Wie in Figur 1 dargestellt, kann durch den Miniatur-Laserprojektor ein Bild auf einen Projektionsort projiziert werden, das alle Informationen enthält, die sonst auch üblicherweise auf Monitoren bzw. Bildschirmen von Navigationssystemen dargestellt werden. Das Projektionsbild selbst ist mit dem Bezugszeichen 14 versehen. Es kann dem behandelnden Arzt alle möglichen Informationen über den Patientenkörper, die derzeitige Patienten- und Instrumentenposition oder geplante Eingriffstrajektorien bieten, wobei der Projektionsort selbst sehr viel freier wählbar ist als die Anordnung von Monitoren gemäß dem Stand der Technik.

Die Figur 2 zeigt eine weitere Ausführungsform ähnlich der Figur 1, wobei jedoch der Laserprojektor 10 zusätzlich mit einer Navigationsreferenz 18 versehen ist. Die Navigationsreferenz 18 wird durch das Navigationssystem 15 positionell geortet und erfasst, und dadurch lässt sich grundsätzlich auch das Bild 14 je nach Lage des Projektors anpassen. Dies kann beispielsweise dann nützlich sein, wenn auf ortsbekannte Projektionsflächen projiziert wird und der Projektor 10 selbst in einem relativ spitzen Winkel dazu steht. Das Bild könnte dann nach Berücksichtigung der Position des Projektors 10 trotzdem so angepasst und ausgegeben werden, als ob es senkrecht aufprojiziert würde.

Eine weitere Möglichkeit der Verwendung eines navigierten Miniatur-Laserprojektors ist in Figur 3 gezeigt. Hier ist schematisch das Instrument 2 mit seinem funktionellen Abschnitt, beispielsweise der Klinge 4 dargestellt. An diesem Instrument 2 ist wiederum fest angebracht oder integriert der Laserprojektor 10 dargestellt, der eine Navigationsreferenz 18 bzw. Ortungsreferenz 18 aufweist. Im vorliegenden Fall projiziert der Projektor 10 auf die Oberfläche eines Patientenkörperteils 6 eine Hilfsdarstellung 8. Der Körperteil 6 ist nur ausschnittsweise dargestellt, und ebenfalls schematisch dargestellt ist eine Navigationsreferenz 7, die fest an dem Körperteil 6 angeordnet ist. Das Körperteil 6 kann z.B. die Schädeloberfläche eines Patienten sein, in die mit der Klinge 4 des Instruments 2 ein Einschnitt gemacht werden soll.

Um dem behandelnden Arzt dabei zu helfen, das Instrument 2 im richtigen Winkel zu führen und an der richtigen Stelle einzuschneiden, können nun durch den Projektor 10 auf das Körperteil 6 Hilfsinformationen aufprojiziert werden, hier konzentrische Kreise 8 mit einem Zentrum 5. Dies ist möglich, weil die Navigationssysteme 15 die Position des Instruments 2 und des Laserprojektors 10 über die Navigationsreferenz 18 bekannt ist, ebenso wie die Position des Patientenkörperteils 6 über den Referenzstern 7.

Es kann also mittels des Projektors 10 eine Bildinformation auf den Patienten aufprojiziert werden, welche den behandelnden Chirurgen bei dem Führen seines Instruments 2 hilft. Sind die Kreise 8 beispielsweise alle konzentrisch, so liegt das Instrument in der richtigen Ausrichtung, werden die Kreise oval und gehen in einer Richtung auseinander, so ist das Instrument in dieser Richtung verkippt. Eine weitere Information ist beispielsweise die Mittellage 5, die den Eingriffspunkt für die Spitze der Klinge 4 anzeigen kann.

Zusätzlich oder anstelle der Hilfsinformationen bzw. Hilfslinien 5, 8 können auch unter der Haut liegende Körperteile aufprojiziert werden, und so kann man dem behandelnden Arzt praktisch ein Navigationssystem liefem, das zur Bilddarstellung einerseits auf Monitore verzichten kann und andererseits alle notwendigen Bilddaten dort darstellt, wo ihr Vorhandensein am Wichtigsten ist, nämlich an der Operationsstelle selbst. Natürlich kann eine solche erfindungsgemäße Laserprojektion auch zusätzlich zu einer Bildschirm- bzw. Monitordarstellung erfolgen.

## Patentansprüche

1. Medizintechnisches Bilddarstellungssystem mit:
a) einer Bildspeicherungs- und Bildverarbeitungseinrichtung (15), die Patientenkörper-Bilddaten und/oder Bildzusatzdaten speichert, verarbeitet und ausgibt; und mit
b) einer tragbaren Laserprojektionsvorrichtung (10), welche die von der Bildspeicherungs- und Bildverarbeitungseinrichtung ausgegebenen Bilddaten und/oder Bildzusatzdaten auf einen Projektionsort projiziert.

2. Bilddarstellungssystem nach Anspruch 1, bei dem die Bildspeicherungs- und Bildverarbeitungseinrichtung ein Teil eines medizintechnisches Navigationssystem (15) ist oder einem medizintechnischen Navigationssystem (15) zugeordnet ist.

3. Bilddarstellungssystem nach Anspruch 2, bei dem die tragbare Laserprojektionsvorrichtung 10) eine Navigationsreferenz (18) aufweist, die von dem Navigationssystem (15) geortet und positionell bestimmt werden kann.

4. Bilddarstellungssystem nach einem der Ansprüche 2 oder 3, bei dem die Laserprojektionsvorrichtung (10), insbesondere abnehmbar, an einem medizinischen Instrument (2) angeordnet ist oder in ein medizinisches Instrument integriert ist.

5. Bilddarstellungssystem nach Anspruch 4, bei dem das medizinische Instrument eine Navigationsreferenz (18) aufweist, die von dem Navigationssystem (15) geortet und positionell bestimmt werden kann.

6. Bilddarstellungssystem nach einem der Ansprüche 1 bis 4, bei dem die Laserprojektionsvorrichtung (10), insbesondere abnehmbar, an einem medizinischen Behandlungsgerät oder einem behandlungsunterstützenden Gerät angeordnet ist oder angeordnet werden kann, insbesondere an einem der folgenden Geräte:
- einem chirurgischen Mikroskop;
- einem Bilderfassungsgerät, z.B. einem Schichtbilderfassungsgerät oder einem C-Bogen-Röntgengerät oder Fluoroskop;
- einem Strahlenbehandlungsgerät (LINAC);
- einer Operationslampe;
- einem Pointer,
- einem invasiven Instrument, z. B. einem Skalpell;
- **einer Biopsie-Nadelhaltevorrichtung;**
- einer Biopsienadel.

7. Medizintechnisches Bilddarstellungsverfahren, bei dem eine Bildspeicherungs- und Bildverarbeitungseinrichtung (15) Patientenkörper-Bilddaten und/oder Bildzusatzdaten speichert, verarbeitet und ausgibt; und bei dem mit einer tragbaren Laserprojektionsvorrichtung (10) die von der Bildspeicherungs- und Bildverarbeitungseinrichtung ausgegebenen Bilddaten und/oder Bildzusatzdaten auf einen Projektionsort projiziert werden.

8. Bilddarstellungsverfahren nach Anspruch 7, bei dem Bilddaten und/oder Bildzusatzdaten projiziert werden, die aus einem medizintechnischen Navigationssystem stammen.

9. Bilddarstellungsverfahren nach Anspruch 7 oder 8, bei dem die Bilddaten Patientenanatomiedaten sind, insbesondere Projektionen aus Schichtbildaufnahmen und Durchleuchtungsaufnahmen.

10. Bilddarstellungsverfahren nach einem der Ansprüche 7 bis 9, bei dem die Bildzusatzdaten Zusatzinformationen zu den Bilddaten und/oder Hilfsdarstellungen umfassen, wie Navigationsanweisungen in Pfeilform oder als Richtungsanzeigen bzw. Ausrichtungsanweisungen.

11. Bilddarstellungsverfahren nach einem der Ansprüche 8 bis 10, bei dem die Bilddaten und/oder die Bildzusatzdaten durch die Laserprojektionsvorrichtung (10), die in einem medizinischen Instrument (2) integriert oder daran angeordnet sein kann, auf einen Patientenkörperteil (6) projiziert werden, wobei sowohl der Patientenkörperteil (6) als auch die Laserprojektionsvorrichtung (10) bzw. das Instrument (2) von dem Navigationssystem (15) geortet und positionell bestimmt werden.

12. Bilddarstellungsverfahren nach einem der Ansprüche 8 bis 11, bei dem die Laserprojektionsvorrichtung in Kombination mit dem Navigationssystem als eine, insbesondere dynamische, Benutzerschnittstelle verwendet wird, insbesondere als virtuelle Eingabeeinheit.

13. Bilddarstellungsverfahren nach einem der Ansprüche 8 bis 12, bei dem die Laserprojektionsvorrichtung einem Videokamera-Trackingsystem zugeordnet wird und die von diesem System erfassten Bildinformationen ausgibt.

14. Bilddarstellungsverfahren nach einem der Ansprüche 8 bis 13, bei dem die Laserprojektionsvorrichtung verwendet wird, um auf eine Körperoberfläche ein bekanntes und diversifizierendes Muster aufzuprojizieren, und bei dem das aufprojizierte Muster von einer Videokamera aufgenommen bzw. (re-)detektiert wird, um aus der Form oder Verformung des Musters zu Registrierungszwecken auf die Form der Körperoberfläche zu schließen.
